Europäisches Patentamt

European Patent Office

Office européen des brevets

(11) Publication number: **0 341 799**
**A2**

## EUROPEAN PATENT APPLICATION

(21) Application number: **89201177.6**

(22) Date of filing: **10.05.89**

(51) Int. Cl.⁴: **G06K 19/06**

(30) Priority: **13.05.88 IT 2056388**

(43) Date of publication of application:
**15.11.89 Bulletin 89/46**

(84) Designated Contracting States:
**AT BE CH DE ES FR GB GR LI LU NL SE**

(71) Applicant: **BELLCO S.p.A.**
**Via Camurana 1**
**I-41037 Mirandola Modena(IT)**

(72) Inventor: **Ferri, Angelo**
**Via F.lli Cairoli 37/B,**
**I-41037 Mirandola, Modena(IT)**

(74) Representative: **Roggero, Sergio et al**
**Ing. Barzanò & Zanardo Milano S.p.A. Via**
**Borgonuovo 10**
**I-20121 Milano(IT)**

(54) **Device for automatically identifying liquid containers, and containers suitable for this purpose.**

(57) A device is described for automatically identifying liquid containers, consisting of a coded element of material opaque to infrared rays which is permanently secured to the container to be identified and is suitably shaped or perforated to carry the impression of a particular binary code, and, connected to a microprocessor, an infrared sensor system into which said coded element can be inserted and which is able to unambiguously read its code. This system, of extreme interest for example in the hemodialysis field, enables the container (and hence the liquid with which it is filled) to be identified by the particular code impressed on the coded element secured to it.

A further objecto of the present invention are the containers equipped with the coded element and its use in hemodialysis field.

FIG. 2

## DEVICE FOR AUTOMATICALLY IDENTIFYING LIQUID CONTAINERS, AND CONTAINERS SUITABLE FOR THIS PURPOSE

This invention relates to a device for automatically identifying liquid containers, consisting of a coded element of material opaque to infrared rays which is permanently secured to the container to be identified and is suitably shaped or perforated to carry the impression of a particular binary code, and an infrared reader connected to an electronic recognition apparatus and into which said coded element can be inserted and which is able to unambiguously read its code. This system enables the container to be identified by the coded element secured to it, with corresponding identification of the particular composition, associated with the particular code, of the liquid with which the container is filled.

This facility is of particular interest in the hemodialysis field, and the invention has been developed precisely as the solution to a problem which arises in this field. The invention is described hereinafter in greater detail with reference to this particular aspect of its application. It is to be understood however that the system of the present invention is of general application. In this respect, with most of the commercially available hemodialysis preparators the dialysis liquid, the composition and concentration of which can vary according to the particular patient and treatment, is automatically monitored in terms of temperature and conductivity to evaluate its suitability for feeding to the dialyser and for contact with the patient's blood. If the liquid temperature or electrical conductivity is not acceptable, an automatic control system for the apparatus automatically halts its operation and signals the cause of the stoppage.

However, the chemical composition of the liquid is not automatically checked, with the result that basically any liquid having a temperature and electrical conductivity between determined limits could be accepted by the apparatus. It should also be noted that during the last decade dialysis techniques have become progressively refined and the types of concentrated solutions used in hemodialysis have now considerably increased in number. In addition, in some methods more than one concentrated saline solution must be used simultaneously (such as in so-called bicarbonate dialysis).

There is therefore the possibility of using unsuitable solutions because of human error.

This possibility of error is not negligible, firstly because for obvious practical and convenience reasons hemodialysis containers are all of a similar shape and size whether they contain hemodialysis solutions of different concentrations or solutions for other uses (disinfectants), secondly because this treatment at hospital level is carried out by personnel not highly qualified, and finally because of the increasing use of domiciliary dialysis. The effect of using the wrong solution obviously varies according to the type of solution and the sensitivity of the patient. The consequences can in any event sometimes be very serious.

The object of the system according to the present invention is to reduce the possibility of error in the use of dialysis solutions to a minimum.

Using the system of the present invention, the container, filled with a determined concentrated solution for hemodialysis, is characterised by the presence of a coded element rigidly and permanently secured to the container during its making-up. This coded element is suitably shaped or perforated to carry the impression of a particular code which can be read and decoded by a suitable infrared reader into which said coded element is inserted before the dialysis session, and which is connected to a microprocessor.

Said coded element can be made of any material which is able to be worked into the desired form, is sufficiently rigid to be able to be inserted into the sensor without deformation, and is opaque to infrared rays.

Materials which can be used for this purpose include, by way of example, metal materials, wood, cardboard and, preferably, all those low-cost plastics materials of conventional use which are opaque to infrared rays. Typical examples of such materials are suitably coloured PVC, polyethylene, polycarbonate etc.

The use of infrared rays (typically in the near infrared range of 0.8-1.0μ) reduces the possibility of error to a minimum. In particular, even if a coded element becomes encrusted with salts from the container solution which may have escaped as it was being filled or during its use, there is no risk of false results, as would be possible with a white-light optical reader.

This coded element can basically have any shape and size provided it can receive the coding and the code can be read by an infrared sensor. Preferably, for practical reasons the coded element is in the form of a small-dimension strip of variable thickness. More preferably, the coded element is in the form of a fairly thin small plate of elongated shape.

The coded element can be secured to the container in any manner, for example it can be an appendix of the container itself, obtained during the moulding of the container, or it can be applied to the container or to a part of it by a metal wire or chain or a strip or ring of the same plastics material as that which may have been used, or in any other possible manner. It is preferable for said coded element secured to the container to be freely positionable to enable it to be more easily introduced into the sensor at the time of identification, and to obviate any storage and stocking problems.

According to a preferred aspect of the invention said coded element is secured to the neck of the container in a manner able to rotate freely about it.

Said coded element becomes an integral part of the container and is thrown away after use together with the container itself. In particular, the coded element is unable to be removed from the container without tampering with the pack.

The code can be applied by disposing suitable perforations in one or more rows or by suitably shaping one or both sides, so that when the coded element has been correctly inserted into the infrared sensor, each of the n possible coding positions is read by an individual reader unambiguously, and the sequence of on-off signals thus obtained can be correctly decoded by the recognition apparatus. The number of reading positions in the infrared sensor, which will be equal to the number of coding positions in the coded element secured to the container, will depend obviously on the number of different concentrated solutions to be coded. For example, 3 reading positions will allow 8 different codes, 4 positions will allow 16 different codes and so on, the presence of n reading positions allowing $2^n$ different codes.

Considering the ever increasing number of dialysis solutions which are investigated and conceived clinically for the purpose of reducing as much as possible the discomfort of patients subjected to this type of treatment, between 3 and 10 coding positions should satisfy all requirements at least at the present time. According to a preferred aspect of the present invention, the coded element is in the shape of a flat bar typically having dimensions of between 3 and 15 cm in length and between 0.5 and 5 cm in width, and containing suitable perforations disposed in a single row, or being shaped (or toothed) along only one of its sides. This in fact enables the production of the sensor unit to be simplified (and its cost reduced) by making it possible to simply sequence a number n of already commercially available individual readers.

If shaping is provided along only one of the two sides, the other side can act as a sort of guide to facilitate insertion of the coded element into the sensor.

The device of the present invention can be provided with further expedients to ensure correct and complete insertion of the coded element into the sensor.

For example, the coded element can be snap-inserted into the sensor to prevent its random escape, with stoppage of the dialysis apparatus. Again, for example the value zero can be allotted for the most inner of the individual readers and an alarm system can be included to halt the apparatus if the infrared ray of this reader is not intercepted by the coded element, because it has been badly inserted.

In all cases, the signal deriving from each reading position of the infrared sensor and converted into an electric signal is fed via a multiwire cable to a microprocessor where the sequence of n on-off signals is decoded and possibly converted into a signal visible on a display to indicate the particular solution used. This apparatus can interact with the hemodialysis preparator in two different ways: the apparatus can either be provided with a blocking system which requires the operator to check the information appearing on the display and then to operate a release device, or be provided with an internal checking system which, when the dialysis treatment schedule has been set, compares the information received by the sensor unit with the set data and if these coincide it automatically releases the apparatus. In the first case, the attention of the operator can be drawn not only by the fact that the apparatus has stopped operation but also by acoustic and/or luminous signals. If the treatment provides for the use of two different saline solutions simultaneously, as in so-called bicarbonate dialysis, the apparatus is provided with two different infrared sensors, one for each container. In this case it is also possible that before visually displaying the type of formulation used, the microprocessor automatically checks the compatibility of the two constituents of the formulation. Again in this latter case a blocking system acts, possibly preceded by acoustic and/or luminous alarms, to request the check to be made by the operator, to ensure correct dialysis treatment.

By way of example, a particular embodiment of this invention is shown in the accompanying drawings.

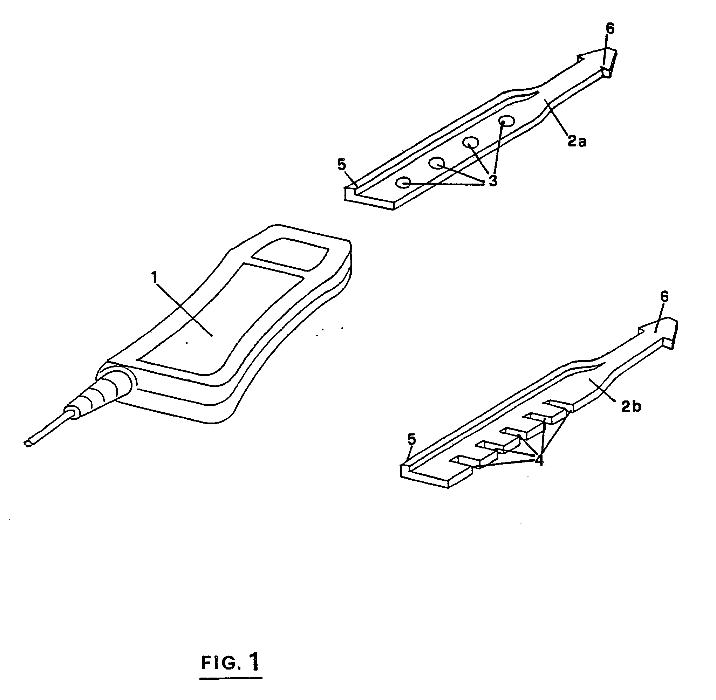

More particularly, Figure 1 shows an external view of the sensor system 1 and two different types of coded elements 2a and 2b in the form of flat strips which in the preferred form 2a has suitable perforations 3 disposed in a single row, or in the form 2b has suitable shaping 4 obtained by simply removing material from one of the two sides. The guide 5 for the correct insertion of the element into the sensor 1 is also shown as is the appendix 6 for securing the element to an eyelet on the container surface.

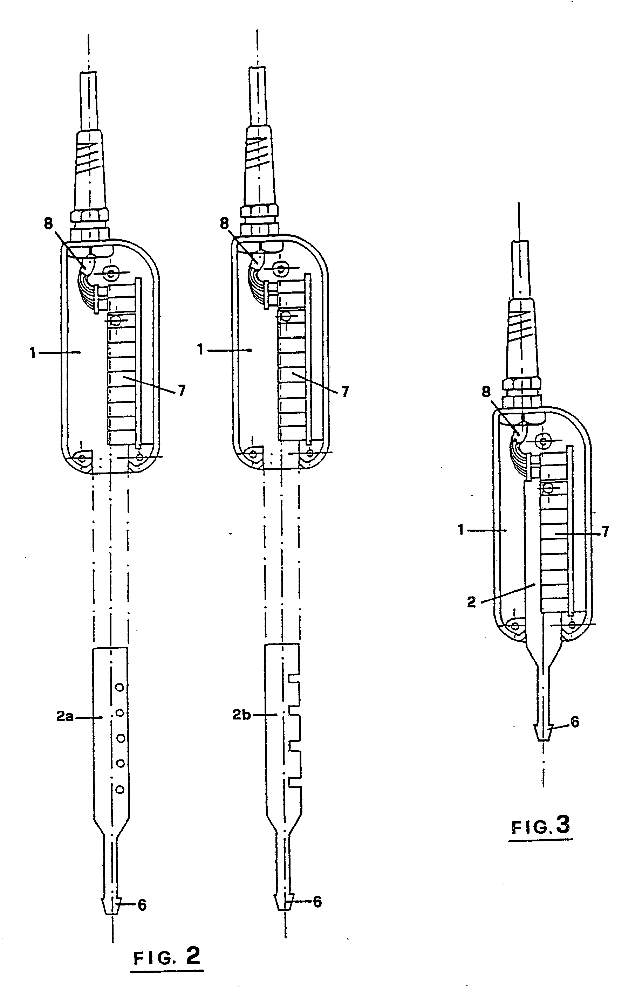

Figure 2 is a front sectional view of the device consisting of the sensor 1 and coded element 2a or 2b respectively, ready for insertion, and

Figure 3 is a front sectional view of the sensor 1 with the coded element 2 inserted. The coded element is indicated by 2a and 2b before insertion and by 2 after insertion. The part 6 which remains outside the sensor is the appendix used for securing the element to the container. The reference numeral 7 indicates the combined succession of individual infrared readers and 8 indicates the multi-wire electric cable which feeds the signals, converted into electric signals, from each reader to the microprocessor.

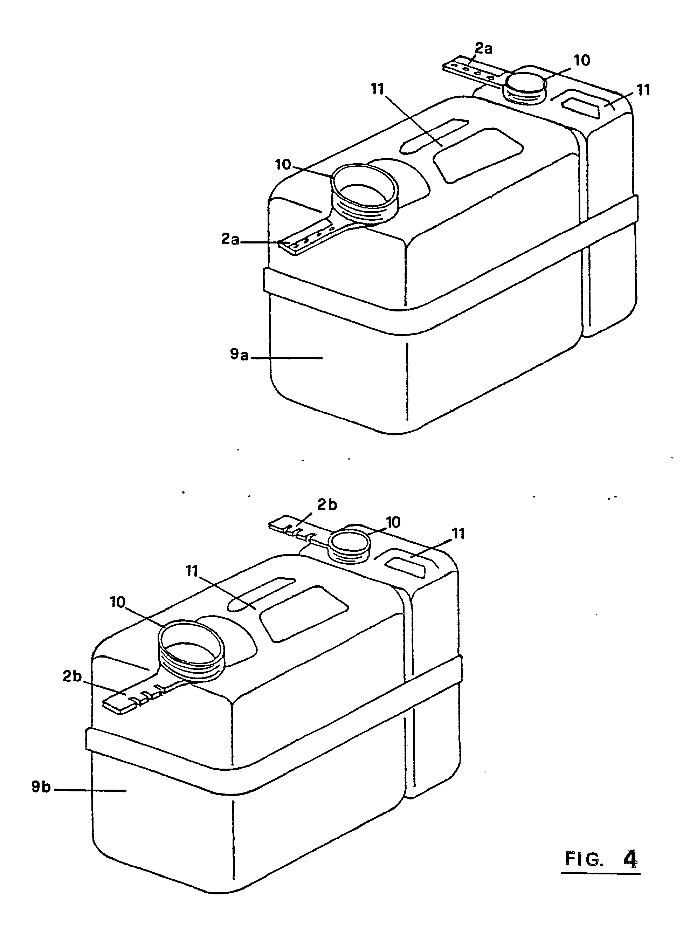

Finally, Figure 4 shows two pairs of containers, of which one, 9a, is provided with the coded element 2a and the other, 9b, is provided with the coded element 2b. In this case the element 2, which is secured to the container neck 10, can rotate about it. These two figures show the coupled container system for dialysis treatment with two solutions. The reference numeral 11 indicates the container handles, to which the elements coded in accordance with the aforesaid methods could be secured as an alternative.

For hemodialysis, the device of the present invention is used as follows:
On switching the machine on without the coded element or elements being inserted into the sensor or sensors, an acoustic and/or luminous alarm system indicates that a first coded element should be inserted into the first sensor and (if the coding of the first container indicates that a second solution has also to be used) that a second coded element should be inserted into the second sensor. If the recognised codes are mutually compatible, they are decoded and the recognised solution or combination of solutions is indicated on the display, otherwise the acoustic and/or luminous alarm system indicates incorrect formulation.

At this point the system requires the confirmation of the operator and remains in a waiting state until he presses the correct key. After this confirmation any movement of the sensors (either by error or to replace empty containers) returns the module to its initial state, to again require code sensing, decoding, visual display and operator confirmation.

Alternatively, once the coded elements are inserted into the sensors, the microprocessor compares the particular decoded solution combination with the set treatment schedule without necessarily providing visual display, and if these concur it allows the apparatus operation to proceed. Again in this case any movement of the sensors returns the module to its initial state. With regard to the control electronics, in practice the microprocessor can be an independent module or can be incorporated directly in the apparatus upstream of the preparator.

## Claims

1. A device for automatically identifying liquid containers, consisting of a coded element of material opaque to infrared rays which is permanently secured to the container to be identified and is suitably shaped or perforated to carry the impression of a particular binary code, and, connected to a microprocessor, an infrared sensor system into which said coded element can be inserted and which is able to unambiguously read its code.

2. A device as claimed in claim 1, wherein said coded element is in the form of a small plate.

3. A device as claimed in claim 2, wherein the code is impressed by perforation along a single row or by shaping only one of the sides of the plate.

4. A device as claimed in claim 3, wherein the coded element contains from 3 to 10 coding positions.

5. A device as claimed in claim 1, wherein the coded element is of plastics material opaque to infrared rays.

6. A device as claimed in claim 1, wherein the coded element is secured to the container but is freely positionable relative to it.

7. A liquid container characterised by having secured in a permanent manner to it a coded element of material opaque to infrared rays and suitably perforated or shaped in such a manner as to carry the impression of a particular binary code associated with a determined composition of the liquid which fills the container, which coded element, when inserted into a suitable infrared reader, can be decoded thereby.

8. A liquid container as claimed in claim 7, wherein the coded element is in the form of a small plate.

9. A container as claimed in claim 8, wherein said small plate is of elongated shape and on which the code is impressed by suitable perforation along a single row or by shaping only one of its sides.

10. A device and container as claimed in any one of the preceding claims, for use in hemodialysis.

11. A method for recognising a hemodialysis solution container, characterised in that the liquid is contained in a liquid container to which there is rigidly secured in a permanent manner a coded element of material opaque to infrared rays and suitably perforated or shaped to carry the impression of a particular binary code associated with a determined composition of the liquid which fills the container, said coded element being inserted into a suitable infrared reader system which is able to detect the code impressed on said coded element and to transmit it to a microprocessor which recognises it.

12. A method as claimed in the preceding claim, wherein the identification device consisting of the coded element and the sensor is as described in any one of claims 2 to 6.

13. A hemodialysis apparatus, characterised by being connected to or incorporating a microprocessor arranged to recognise hemodialysis solution containers in accordance with the method of claim 11.

FIG. 1

FIG. 2

FIG. 3

FIG. 4